# EUROPEAN PATENT SPECIFICATION

(11) **EP 1 230 373 B1**
(45) Date of publication and mention of the grant of the patent: **07.02.2007**
(21) Application number: 00976522.3
(22) Date of filing: 10.11.2000
(51) Int. Cl.: C12N 9/10

(54) **USE OF A CLASS OF ENZYMES AND THEIR ENCODING GENES TO INCREASE THE OIL CONTENT IN TRANSGENIC ORGANISMS**
VERWENDUNG EINER ENZYMKLASSE UND IHRER KODIERENDEN GENE ZUR ERHÖHUNG DES ÖLGEHALTS IN TRANSGENEN ORGANISMEN
UTILISATION D'UNE CLASSE D'ENZYMES ET DE LEURS GENES CODANTS POUR AUGMENTER LA TENEUR EN HUILE D'ORGANISMES TRANSGENIQUES

(30) Priority: 12.11.1999 EP 99850169; 12.11.1999 US 164859 P
(43) Date of publication of application: 14.08.2002
(73) Proprietor: BASF Plant Science GmbH, 67056 Ludwigshafen (DE)
(72) Inventor: BANAS, Antoni, PL-08-110 Siedlce (PL); SANDAGER, Line, DK-2100 Copenhagen (DK); STAHL, Ulf, S-753 24 Uppsala (SE); DAHLQVIST, Anders, S-244 66 Furulund (SE); LENMAN, Marit, S-223 59 Lund (SE); RONNE, Hans, S-756 54 Uppsala (SE); STYMNE, Sten, S-268 90 Svalöv (SE)
(74) Representative: Aldenbäck, Ulla Christina
(86) International application number: PCT/SE2000/002216
(87) International publication number: WO 2001/034814

(56) References cited:
- WO-A1-97/45439
- WO-A1-98/55631
- WO-A1-99/63096
- DATABASE MEDLINE [Online] OELKERS P. ET AL.: 'Characterization of two human genes encoding acyl coenzyme A: Cholesterol acyltransferase-related enzymes', XP002951401 Retrieved from Dialog Information Services, File 155, accession no. 09679766 Database accession no. 98434592 & JOURNAL OF BIOLOGICAL CHEMISTRY vol. 273, no. 41, 09 October 1998, (UNITED STATES), pages 26765 - 26771
- HONGYUAN YANG ET AL.: 'SterolEsterification in yeast: A two-gene process' SCIENCE vol. 272, May 1996, pages 1353 - 1356, XP002937218
- KATHRYN LARDIZABAL ET AL.: 'Identification of a new diacylglycerol acyltransferase gene family' NPLC SYMPOSIA ON BIOCHEMISTRY AND MOLECULAR BIOLOGY OF PLANTGLYCEROLIPID BIOSYNTHESIS, [Online] June 1999, SOUTH LAKE TAHOE, CALIF., XP002937217 Retrieved from the Internet: <URL:http://www.msu.edu/user/ohlrogge/Abstr acts99z.html>
- DATABASE MEDLINE [Online] CASES S. ET AL.: 'Identification of a gene encoding an acyl CoA: Diacylglycerol acyltransferase, a key enzyme in triacylglycerol synthesis', XP002951402 Retrieved from Dialog Information Services, File 155, accession no. 09690408 Database accession no. 99007259 & PROC. NATL. ACAD. SCI. USA vol. 95, no. 22, 27 October 1998, pages 13018 - 13023

## Description

### FIELD OF THE INVENTION

The present invention relates to the use of a novel enzyme and its encoding gene for transformation. More specifically, the invention relates to the use of a gene encoding an enzyme with acyl-CoA : diacylglycerol acyltransferase activity. This gene expressed alone in transgenic organisms will increase the total amount of oil (*i.e.* triacylglycerols) that is produced.

### BACKGROUND OF THE INVENTION

In oil crops like rape, sunflower, oilpalm etc., the oil (*i.e.* triacylglycerols) is the most valuable product of the seeds or fruits and other compounds such as starch, protein and fiber is regarded as by-products with less value. Enhancing the quantity of oil per weight basis at the expense of other compounds in oil crops would therefore increase the value of the crop. If enzymes regulating the allocation of reduced carbon into the production of oil can be upregulated by overexpression, the cells will accumulate more oil at the expense of other products. This approach could not only be used to increase the oil content in already high oil producing organisms such as oil crops, they could also lead to significant oil production in moderate or low oil containing crops such as soy, oat, maize, potato, sugar beats, and turnips as well as in microorganisms.

Development in genetic engineering technologies combined with greater understanding of the biosynthesis of triacylglycerols now makes it possible to transfer genes coding for key enzymes involved in the synthesis of triacylglycerols from a wild plant species or organisms of other kingdoms into domesticated oilseed crops. In this way, triacylglycerols can be produced in high purity and quantities at moderate costs.

It is known that the biosynthesis of triacylglycerols (TAG) in fat-accumulating tissues in animals (Bell & Coleman, 1983) and plants (Cao & Huang, 1986, Martin & Wilson 1983) as well as the accumulation of oil in microbial organisms such as bacteria (Ekundayo & Packter, 1994), yeast and other fungi (Ratledge 1989) can be catalyzed by acyl-CoA : diacylglycerol acyltransferases (DAGATs), enzymes that transfer an acyl-group from acyl-CoA to diacylglycerol, thus forming TAG.

During the past few years genes coding for DAGATs, have been identified in animals (Cases et al., 1998), plants (Hobbs et al., 1999; Lardizabal et al., 2000) and in microbes (Lardizabal et al., 1999). These DAGATs belong to two unrelated protein families.

The first type of DAGAT that was characterized, DAGAT A, has so far been found only in animals (Cases et al., 1998) and plants (Hobbs et al., 1999). These genes show sequence similarities to genes encoding acyl-CoA : cholesterol acyltransferase (ACAT). The mouse DAGAT A has 20 % amino acid sequence identity to the mouse ACAT (Cases et al., 1998). However, DAGATs A from plants and animals are more similar to each other than to ACAT. Thus, the mouse DAGAT A has 38 % amino acid sequence identity to the *Arabidopsis thaliana* DAGAT A (Hobbs et al., 1999). It is also approximately 80% identical to the human ACAT like protein ARGP1, which was suggested to be involved in TAG synthesis (Oelkers et al., 1998), indicating that ARGP1 is a DAGAT A.

The yeast *S. cerevisiae* contain 2 genes with sequence similarity to ACAT, *ARE1* and *ARE2* (Yang et al., 1996). The encoded proteins have approximately 24 % overall amino acid sequence identity to the mouse ACAT and 15% identity to the DAGAT A from mouse. It should be noted that they are both more similar to each other (45% amino acid sequence identity) than to either ACATs or DAGATs from higher eukaryotes. It is not possible to classify them as putative ACATs or DAGATs based on sequence similarities alone, since their evolutionary distances from both groups of higher eukaryotic enzymes are similar. However, experiments have shown that both Are1 and Are2 are ACATs, which together are responsible for all of the sterol ester synthesis that occurs in yeast (Yang et al., 1996; Yu et al., 1996). The possible involvement of Are1 and Are2 in the synthesis of TAG has also been studied (Yang et al., 1996; Yu et al., 1996). From these studies, it was concluded that Are1 and Are2 are not involved in TAG synthesis. Thus, there is no prior art to show that Are1 is a TAG synthesizing enzyme, nor can it be concluded, on the basis of homologies to ACAT like sequences already published, that Are1 is a DAGAT (Lassner and Ruezinsky, 1999).

The second family of DAGAT enzymes, the DAGAT B family, is unrelated to any other known proteins. These enzymes show no sequence homology to the mouse and plant ACAT like DAGAT A proteins (Lardizabal et al., 2000) or to any other known proteins.

DAGAT A and B are not the only enzymes that contribute to TAG biosynthesis. TAG can also be synthesized by an acyl-CoA independent reaction. Thus, the newly discovered enzyme phospholipid : diacylglycerol acyltransferase (PDAT) catalyses the formation of TAG by transferring an acyl group from the sn-2 position of a phospholipid to DAG (Dahlqvist et al., 1999; Ståhl, 1999).

### SUMMARY OF INVENTION

This invention describes the identification of a gene encoding an enzyme that is partly responsible for TAG accumulation in yeast.

In one aspect, this invention relates to the use of this gene in recombinant DNA constructs to direct the transcription and translation of the diacylglycerol acyltransferase sequence of the present invention in a host organism or progeny thereof, such as an oil-producing plant. Cells and organisms containing the diacylglycerol acyltransferase as a result of the production of the acyltransferase encoding sequence are also included within the scope of the invention.

Of particular interest is the expression of the nucleotide sequences of the present invention from transcription initiation regions that are preferentially expressed in plant seed tissues. It is contemplated that the gene sequence may be synthesized, especially when there is interest to provide plant-preferred codons.
In a different embodiment, this invention also relates to methods of using a DNA sequence encoding a said protein of the present invention for increasing the oil-content within the cells of different oil-producing plants.

Further, the invention makes possible a process for the production of triacylglycerol, which comprises growing transgenic cells or organisms under conditions whereby any of the nucleotide sequences discussed above are expressed in order to produce an enzyme in these cells with the ability to transfer a fatty acid from acyl-CoA to diacylglycerol, thus forming triacylglycerol.

The present invention can be essentially characterized by the following aspects:
1. Use of a nucleic acid sequence encoding an enzyme catalysing the transfer of a fatty acid from acyl-CoA to diacylglycerol for the production of triacylglycerol (TAG) by genetic transformation of an oil-producing organism with said sequence in order to be expressed in this organism and result in an active enzyme in order to increase the oil content of the organism.
   The nucleic acid sequence is derived from the sequence shown in SEQ ID NO. 1, from the *Saccharomyces cerevisiae ARE1* gene (genomic clone or cDNA), or from a nucleic acid sequence or cDNA that contain nucleotide sequences coding for a protein with an amino acid sequence that is 60% or more identical to the amino acid sequence as presented in SEQ. ID. NO. 2.
2. Transgenic organisms comprising, in their genome or on a plasmid, a nucleic acid sequence according to the above, transferred by recombinant DNA technology. The transgenic organisms are selected from the group consisting of fungi, plants and animals. Preferably the transgenic organisms agricultural plants and preferably said nucleotide sequence is expressed under the control of a storage organ specific promoter. Alternatively, the nucleotide sequence is expressed under the control of a seed-specific promoter.
3. A protein encoded by a DNA molecule according to SEQ ID NO. 1 or a functional (enzymatically active) fragment thereof. Alternatively, the protein produced in an organism as specified in aspect 2, which has the amino acid sequence set forth in SEQ ID NO. 2 or an amino acid sequence with at least 60 % homology to said amino acid sequence. Preferably the protein is isolated from *Saccharomyces cerevisiae.*
4. Use of a protein as specified in aspect 3 in the production of triacylglycerols.

### DETAILED DESCRIPTION OF THE INVENTION

The invention now having been generally described will be more readily understood by reference to the following drawings and examples, which are included for the purpose of illustration only, and are not intended to limit scope of the present invention.

### Description of the figures:

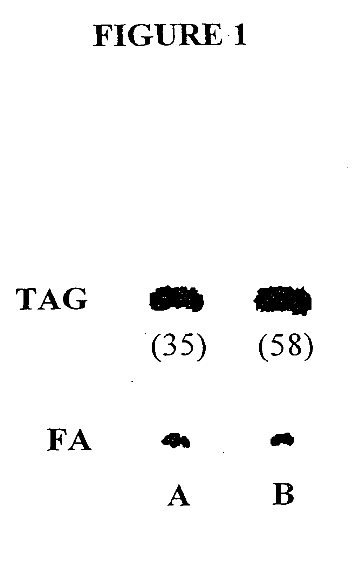

**Figure 1. *In vitro* DAGAT activity in a yeast strain (SCY62) that overexpresses the *ARE1* gene.** Aliquots of microsomal membranes prepared from the control strain (lane A) or the *ARE1* overexpressing strain (lane B) were assayed for DAGAT activity according to Method A described in Material and Methods. The radioactive triacylglycerol synthesised was visualised and quantified as cpm (figures in brackets) on the TLC plate by electronic autoradiography (Instant Imager, Packard, US). Abbreviations used in the figure:
   triacylglycerol (TAG) and unesterified fatty acids (FA).

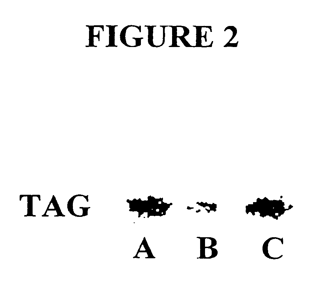

**Figure 2. *In vitro* DAGAT activity in a PDAT DAGAT B double mutant, a PDAT DAGAT B *ARE1* triple mutant, and in the same triple mutant containing a plasmid that overexpresses the *ARE1* gene.**
   The radioactive triacylglycerols (TAG) synthesised in microsomes from the double mutant, H1226 (lane A), the triple mutant, H1236 (lane B) and the same triple mutant containing a plasmid that overexpresses the *ARE1* gene (lane C) were visualised on a TLC plate by electronic autoradiography (Instant Imager, Packard, US).

### BRIEF DESCRIPTION OF THE SEQ ID:

SEQ ID NO. 1: Genomic DNA sequence of the *Saccharomyces cerevisiae ARE1* gene , ORF YCR048W.

SEQ ID NO. 2: The amino acid sequence of the open reading frame YCR048W from *Saccharomyces cerevisiae.*

### EXAMPLES

### EXAMPLE 1 - Triacylglycerol accumulation is reduced in yeast cells that lack the ARE1 gene

### Materials and Methods

**Yeast strains.** Yeast strains used in this study were congenic to the W303-1A (Thomas & Rothstein, 1989) background. An *are1* mutant strain, H1111, with the genotype *MATα are1-Δ::HIS3 ADE2 can 1-100 leu2-3,112 trp1-1 ura3-1,* was generated by crossing the two strains SCY60 *(MATα are1-Δ::HIS3 ade2-1 can 1-100 leu2-3,112 trp1-1 ura3-1*) and SCY61 *(MAT***a** *are2-Δ::LEU2 ADE2 can 1-100 his3-11,15 trp1-1 ura3-1*) (Yang et al., 1996) and dissecting tetrads. As a wild type control, we used SCY62 *(MAT***a** *ADE2 can 1-100 his3-11,15 leu2-3 trp1-1 ura3-1)* (Yang et al., 1996). Yeast mutant strains disrupted in YNR008w and YOR245c encoding yeast DAGAT B and PDAT, respectively, and the *ARE1* gene were constructed through a series of yeast transformations using the lithium acetate method. Linear DNA fragments used for the disruption of the YOR245c and YNR008w genes were created as follows. Primers specific for YOR245c (300 bases upstream, CAGCATTGACGTAATGGGAA, and downstream, AAAGCCAAAAAGAGAAGGACA, of the gene) were constructed and the gene was synthesised using PCR from SCY62 genomic DNA. The PCR-fragment was blunt-ended and ligated into pUC119 previously cleaved with the restriction enzyme *Sma*I. The resulting plasmid, YOR245c-pUC119, was then digested with *Cla*I/*Stu*I and dephosphorylated to prevent religation. The marker KanMX4 was obtained by digestion of the plasmid pFA6a by *SmaI*/*SacI.* The blunted KanMX4 fragment was then ligated into the YOR245c-pUC119 vector between the *Cla*I and *Stu*I sites within the YOR245c open reading frame. A linear fragment containing the resulting YOR245c::KanMX4 disruption cassette was finally obtained through cleavage by *Bam*HI/*Nde*I. The linear fragment used to disrupt the YNR008w gene was constructed in a similar manner as the YOR245c::KanMX4 fragment. The YNR008w gene was amplified from SCY62 genomic DNA, cloned into the pBluescript vector (Dahlqvist et al., 2000) and digested with restriction enzyme *Bbs*I/*Mun*I*.* The *TRP1* marker was then ligated between the *Bbs*I and *Mun*I sites in the YNR008w-pBluescript plasmid, and a linear fragment containing the disruption cassette was obtained by *Bam*HI/*Pst*I digestion. The single PDAT mutant, H1079, with the genotype MATa *pdat-Δ::TRP1 ADE2 leu2-3,112 ura3-1 his3-11,15 trp1-1,* was generated by transforming the wild type strain SCY62 with the linear YNR008w::TRP1 fragment. The PDAT DAGAT B double mutant, H1226, with the genotype MATa *pdat-Δ::TRP1 dagat B-Δ::KanMX4 ADE2 leu2-3,112 ura3-1 his3-11,15 trp1-1,* was constructed in an identical manner by transforming H1079 with the linear YOR245c::KanMX4 fragment. An *ARE1* PDAT double mutant, H1224, with the genotype MATα *are1-Δ::HIS3 pdat-Δ::TRP1 ADE2 can 1-100 leu2-3,112 ura3-1 trp1-1,* was generated by transforming H1111 with the linear YNR008w::TRP1 fragment. The triple mutant strain, H1236, with the genotype MATα *are1-Δ::HIS3 pdat-Δ::TRP1 dagat B-Δ::KanMX4 ADE2 leu2-3,112 ura3-1 trp1-1,* was constructed by transforming H1224 with the linear YOR245c::KanMX4 fragment.

**Yeast Cultivations.** Yeast cells were cultivated at 28 or 30°C on a rotary shaker in liquid YPD medium (1% yeast extract, 2% peptone, 2% glucose). Transformed cells were grown in synthetic medium (Sherman et al., 1986) lacking uracil and supplemented with 2 % (vol/vol) glycerol and 2% (vol/vol) ethanol.

**Lipid Analysis.** The lipid content of the yeast cells was determined as described by Dahlqvist et al. (2000) and is presented as nmol of fatty acid (FA) per mg dry weight yeast.

### Results

The lipid content of a mutant yeast strain (SCY60), in which the *ARE1* gene was disrupted, was analyzed and compared to wild type yeast cells (SCY62) at different stages of growth. In *are1* mutant cells, harvested in exponential phase after 10 hours of cultivation, the total amount of lipid, measured as nmol FA per dry weight yeast, was not significantly different from the wild type yeast (table 1), nor did the amount of fatty acids accumulated into TAG differ strongly between the wild-type and the *are1* mutant. The effect of the *are1* disruption on oil accumulation in stationary phase cells was analysed in an experiment were the yeast cells were pre-cultivated for 24 h in liquid YPD medium. The cells were then harvested and re-suspended in minimal medium (Meesters et al, 1996), supplemented with 16 g/l glycerol, to the original volume of the growth culture. In this glycerol supplemented minimal medium the yeast cells will enter stationary phase under conditions suitable for TAG accumulation. After further cultivation for 24 h, the cells were harvested and their lipid composition was determined. The total lipid content in the *are1* mutant was 15% less than in the wild type. The TAG amount in the *are1* mutant was almost 40 % lower than in the wild type, whereas the polar lipid content did not differ significantly between the *are1* mutant and the wild type yeast (table 1).

Two other genes, YNR008w and YOR254c (Ståhl, 1999; Dahlqvist, et al., 2000; Lardizabal et al., 2000) have recently been shown to be involved in TAG synthesis in yeast. These genes encode a PDAT and a DAGAT B protein, respectively. A yeast strain disrupted in all three genes (ARE1, YNR008w and YOR254c) and a yeast strain with disruptions in only the PDAT and DAGAT B genes were made and they are here named the triple and double mutant, respectively. The TAG content of the double mutant was 48 % of the wild type (table 2), whereas the amount of TAG accumulated in the triple mutant was only 4% of the level in the wild type yeast. By comparing the amounts of TAG accumulated in the double and triple mutants it is clear that Are1 protein contributes to TAG synthesis in yeast.

In summary, these experiment clearly show that the product of the *ARE1* gene contributes to TAG accumulation in yeast.

**Table 1. *Lipid content in ARE1 mutant (SCY60) and wild type (SCY62) yeast cells.*** The lipid accumulation in yeast disrupted in the *ARE1* gene (*are1* mutant) was analysed at different stages of growth and compared to the control wild type yeast. The lipid composition of cells in exponential growth was analysed after 10 hours of cultivation in YPD medium at 28 °C. Yeast cells in stationary phase was prepared by pre-cultivating the cells on liquid YPD medium for 24 hours at 28 °C, after which the cells were harvested, re-suspended in minimal medium (Meesters et al, 1996) supplemented with 16 g/l glycerol, and cultivated for an additional 24 hours at 28 °C. The content of sterol esters, TAG, other neutral lipids, and polar lipids was determined as nmol fatty acids (FA) per mg of dry yeast weight.

| | **SCY62** | | **SCY60** | |
|---|---|---|---|---|
| | (nmol FA / mg) | | (nmol FA / mg) | |
| | **10h** | **48h** | **10h** | **48h** |
| **Sterol esters** | 15 | 24 | 12 | 19 |
| **Triacylglycerol** | 6 | 44 | 8 | 28 |
| **Other neutral lipids** | 4 | 6 | 4 | 5 |
| **Polar lipids** | 65 | 74 | 63 | 74 |
| **Total lipids** | 90 | 148 | 87 | 126 |

### Table 2. Lipid content in the PDAT DAGATB double mutant strain (H1226), in the PDAT

***DAGATB ARE1 triple mutant strain (H1236) and in wild type yeast cells (SCY62).*** The different yeast strains, all of which contained the empty expression plasmid pJN92 (Ronne et al., 1991), were cultivated in YNB medium to which 2 % (v/v) of galactose was added at an A₆₀₀ of 4. The cells were harvested after an additional 22 hours growth and the content of sterol esters, TAG, other neutral lipids, and polar lipids was determined as nmol fatty acids (FA) per mg of dry yeast weight.

| | **SCY62** | **H1226** | **H1236** |
|---|---|---|---|
| | (nmol FA / mg) | (nmol FA / mg) | (nmol FA / mg) |
| **Sterol esters** | 13 | 10 | 1 |
| **Triacylglycerol** | 163 | 78 | 7 |
| **Other neutral lipids** | 17 | 16 | 41 |
| **Polar lipids** | 58 | 66 | 44 |
| **Total lipids** | 251 | 170 | 87 |

### EXAMPLE 2 - Triacylglycerol accumulation is increased in yeast cells that overexpress the ARE1 gene.

### Material and Methods

For induced overexpression of the *ARE1* gene, a 2001 bp *Ehe*I/*Ecl*136II fragment from the plasmid YEP 3-16 (Yang et al., 1996) was cloned into the *Bam*HI site of the *GAL1* expression vector pJN92 (Ronne et al., 1991), thus generating pUS5. The wild type yeast strain SCY62 *(MAT***a** *ADE2 can 1-100 his3-11,15 leu2-3 trp1-1 ura3-1)* (Yang et al., 1996), was transformed with the pUS5 and cultivated at 28 °C on a rotary shaker in synthetic medium (Sherman et al., 1986) lacking uracil and supplemented with 2 % (vol/vol) glycerol and 2 % (vol/vol) ethanol. The *GAL1* promoter was induced after 43 h of growth by the addition of 2 % (wt/vol) final concentration of galactose. Cells were harvested after an additional 24 hours of growth. Wild type (SCY62) cells transformed with the empty vector, pJN92, and cultivated under identical conditions were used as a control. The lipid content of the yeast cells was determined as described by Dahlqvist et al. (2000) and is presented as nmol of fatty acid (FA) per mg dry weight yeast.

**Result**s

The effect of overexpression of the *ARE1* gene on lipid accumulation was studied by transforming the wild-type yeast (strain SCY62) with a plasmid containing the *ARE1* gene under control of the galactose-induced *GAL1* promotor (Table 3). Overexpression of the *ARE1* gene from this promoter had no strong effect on the growth rate as determined by optical density measurements. However, the total lipid content in yeast cells that overexpressed *ARE1* was 1.4 fold higher than in the control yeast transformed with an empty expression vector (Table 3). The elevated lipid content in yeast cells overexpressing *ARE1* is mostly due to a 50% increase in the TAG content, but the amount of sterol esters also increased significantly in these cells, as compared to the control. These results clearly demonstrate that the gene product of *ARE1,* in addition to its earlier reported involvement in the synthesis of sterol esters (Yang et al., 1996), also is involved in TAG synthesis. The elevated levels of TAG achieved in the *ARE1* overexpressing cells also clearly demonstrate the potential use of the *ARE1* gene in increasing the oil content in transgenic organisms.

**Table 3. Lipid content in yeast cells that overexpress the ARE1 gene. Yeast cells (SCY 62) transformed with the ARE1 gene under the control of the GAL1 promotor in the pJN92 vector were cultivated as described in the Material and Method section. Yeast cells (SCY62), transformed with an empty vector, cultivated under identical conditions were used as control. The cells were harvested and the content of sterol esters, triacylglycerols, other neutral lipids and polar lipids was determined as nmol fatty acids (FA) per mg dry yeast weight.**

| | **SCY62** | **SCY62 overexpressing *ARE1*** |
|---|---|---|
| | (nmol FA / mg) | (nmol FA / mg) |
| **Sterol esters** | 19 | 27 |
| **Triacylglycerol** | 160 | 239 |
| **Other neutral lipids** | 30 | 32 |
| **Polar lipids** | 48 | 56 |
| **Total lipids** | 257 | 354 |

### EXAMPLE 3 - The ARE1 gene product has diacylglycerol acyltransferase activity.

### Materials and Methods

*In vitro* diacylglycerol acyltransferase (DAGAT) activity was analyzed, in microsomal fractions prepared from yeast cells, by using one of the following methods.

**Method A:** A wild type yeast (strain SCY62) was transformed with a plasmid (pUS5) containing the *ARE1* gene under the control of a *GAL1* promotor (described in Material and Methods in Example 2). The transformed yeast was cultivated at 28°C in defined YNB medium lacking uracil. The expression of the *ARE1* gene was induced by the addition of 2 % (v/v) galactose after 8 hours growth and the cells were harvested after an additional 17 hours. Microsomal membranes were prepared from the transformed yeast by resuspending 1g of yeast (fresh weight) in 8 ml of ice-cold buffer (20 mM Tris-Cl, pH 7.9, 10 mM MgCl₂, 1 mM EDTA, 5 % (v/v) glycerol, 1 mM DTT, 0.3 M ammonium sulphate) in a 12 ml glass tube to which 4 ml of glass beads (diameter 0.45 - 0.5 mm) were added. The glass tube was heavily shaken (3 x 60 s) with a MSK cell homogenizer (B. Braun Melsungen AG, Germany). The suspension was centrifuged at 20 000 g for 15 min at 6 °C and the resulting supernatant was centrifuged at 100 000g for 2 h at 6 °C. The resulting pellet, containing microsomal membranes, was resuspended in 0.1 M K-phosphate (pH 7.2) buffer and stored at -80 °C. DAGAT activity was analyzed in aliquots of microsomal membranes (50 µl), corresponding to 10 nmol phosphatidylcholine, to which 1 µmol of dioleoyl-PG and 0.25 µmol of dioleoyl-DAG emulsified in 50 µl of buffer containing 190 mM HEPES-NaOH, pH 7.5, 125 mM MgCl₂, 30 mM CHAPS, 2.5 mg/ml BSA and 2 nmol [¹⁴C]-palmitoyl-CoA (2775 dpm/nmol), were added. The reaction mixture was incubated at 30°C for 30 min. The lipids were then extracted in chloroform and separated using thin layer chromatography on silica gel 60 plates in hexane / diethyl ether / acetic acid (80:20: 1). The radioactive lipids were visualized and quantified on the plates by electronic autoradiography (Instant Imager, Packard, US).

**Method B:** The PDAT DAGAT B double mutant (H1226) and the PDAT DAGAT B *ARE1* triple mutant (H1236), described in Material and Methods in Example 1, were transformed with the empty expression plasmid (pJN92). A transformant expressing the *ARE1* gene under the control of the *GAL1* promotor was generated by transforming the triple mutant H1236 with the plasmid pUS5 (described in Material and Methods in Example 2). All yeast transformants were cultivated in YNB medium to which 2 % (v/v) of galactose was added at an A₆₀₀ of 4. The cells were harvested after an additional 6 hours growth and microsomes were prepared using a modification of the procedure of Dahlqvist et al. (2000). Yeast cells (0.2 g) were resuspended in 1.5 ml of ice-cold buffer (20 mM Tris·Cl pH 7.9, 10 mM MgCl₂, 1 mM EDTA, 5 % (vol/vol) glycerol, 1 mM DTT, 0.3 M ammonium sulfate) in a 2 ml Eppendorf tube containing 0.2 ml glass beads (0.45-0.5 mm in diameter). The tube was heavily shaken (3 x 60 s) in a cell homogenizer (Mini Bead Beater). The homogenized yeast was centrifuged at 1350 x g for 20 min at 4 °C, and the resulting supernatant was subsequently centrifuged at 150 000 x g for 1 h at 4 °C. The pellet was re-suspended in 0.1 M potassium phosphate (pH 7.2), and stored at -80°C. Dihexanoyl-DAG (5 nmol) dissolved in chloroform was added to micro tubes and the chloroform was evaporated under a stream of N₂. Aliquots (90 µl) of microsomal fractions corresponding to 150 µg protein, in a buffer consisting of 50 mM HEPES (pH 7.2), 5 mM MgCl₂, and 1 mg/ml BSA were added to the tubes and the suspension was thoroughly mixed. Finally, 10 µl of [¹⁴C]-palmitoyl-CoA (20 nmol, 5000 dpm/nmol) was added, and the mixtures were incubated at 30 °C for 15 min. Lipids were extracted from the reaction mixture into chloroform (Bligh & Dyer, 1959) and separated by TLC on silica gel 60 plates (Merck). The TLC plate was first developed in chloroform / methanol / acetic acid / water (85:15:10:3.5) for 80 mm. The dried plate was then developed in hexane / diethyl ether / acetic acid (80:20:1.5) for 180 mm. The radioactive lipids were visualized and quantified on the plates by electronic autoradiography (Instant Imager, Packard).

### Results

Microsomal membranes prepared from the transformed yeast overexpressing the *ARE1* gene and from control yeast transformed with an empty plasmid (pJN92) were assayed for DAGAT activity according to Method A in Materials and Methods. The amount of radiolabelled TAG synthesized from [¹⁴C]palmitoyl-CoA in microsomal membranes prepared from the *ARE1* overexpressor was increased with 66 % as compared to the control yeast (Fig 1). DAGAT activity was also assayed in microsomal membranes prepared from the PDAT DAGAT B double mutant strain (H1226) and the PDAT DAGAT B *ARE1* triple mutant strain (H1236) cells (Method B). In the double mutant, with a functional *ARE1* gene, TAG with two hexanoyl and one [¹⁴C]palmitoyl chain, was synthesized from added dihexanoyl-DAG and [¹⁴C]palmitoyl-CoA. This synthesis was barely detectable in the triple mutant (figure 2) where the *ARE1* gene was disrupted. However, the in vitro synthesis of TAG was restored in triple mutant cells transformed with a plasmid expressing the *ARE1* gene. This clearly shows that the *in vitro* synthesis of TAG in these yeast mutants correlates with the presence of a functional *ARE1* gene and that the protein encoded by the *ARE1* gene possesses DAGAT activity.

### EXAMPLE 4 -Triacylglycerol accumulation is increased in the seeds of Arabidopsis thaliana that express the ARE1 gene.

### Material and methods

The *ARE1* gene was amplified from the yeast genome using the proof reading enzyme polymerase pfu (Promega). An *Eco*R1 and *Xba*1 restriction enzyme site was introduced respectively into the 5' and 3' ends of this fragment to allow directional cloning of the fragment. The PCR fragment was cloned into the vector pBluescript (Stratagene). The insert derived from this plasmid was then cloned downstream of a napin promoter fragment (Stålberg *et al.,* 1993) in the vector pPGTV-KAN (Becker *et al.,* 1993). This plasmid was transformed into *Agrobacterium* strain GV3301. Transformed *Agrobacterium* cells were then used to transform root explants from *Arabidopsis thaliana* (Valvekens *et al.,* 1992). The lipid content in *Arabidopsis* seeds was determined by methylation of fatty acids. Fatty acids in the oil of proximately 2-3 mg of seeds were methylated in 2 ml 2 % (vol/vol) H₂SO₄ in dry methanol for 90 min at 90°C. The fatty acid methyl esters were extracted with hexane and analyzed by GLC through a 50 m x 0.32 mm CP-Wax58-CB fused-silica column (chrompack), methylheptadecanoic acid was used as internal standard.

### Results

*A. thaliana* was transformed with the *ARE1* gene under the control of a napin promoter, which is seed specific and active during the major phase of oil accumulation. The oil content was analyzed in seeds from single T2 plants derived from four independent transformation events (Table 4). The results showed that in three lines between 50 % and 100 % of the T2 plants generated seeds with statistically significant elevated oil content as compared to the oil content in the seeds from the control plants. The oil content was elevated with up to 18 % in the seeds expressing *ARE1.* One line (28-1) had the same oil content as the seeds from the control plants.

**Table 4. Accumulation of oil in seeds from Arabidopsis thaliana transformed with the ARE1 gene.**

| T2 plants transformed with the *ARE1* gene under the control of the napin promotor and control plants transformed with an empty vector were cultivated in a growth chamber under controlled conditions. The oil content in mature seeds of these plants was determined by GLC analyses and is presented as nmol fatty acids (FA) per mg seed. | | | | | |
|---|---|---|---|---|---|
| | **Transformants** | | | | |
| | **control** | **28-1** | **28-2** | **28-3** | **28-4** |
| Number of T2 plants analyzed | 4 | 6 | 2 | 6 | 11 |
| Number of T2 plants with significant increased seed oil content* | - | 0 | 2 | 3 | 9 |
| nmol FA per mg seed in T2 plant with highest oil content | 1535±114 | 1562±28 | 1753±53 | 1641±82 | 1818±18 |

| | | | | | |
|---|---|---|---|---|---|
| * Calculated with the mean difference two-sided test at α = 5 and based on the average oil content of 4 control plants. | | | | | |

### SEQUENCE LISTING

1 GENERAL INFORMATION
   i) APPLICANT: Scandinavian Biotechnology Research AB
   ii)TITLE OF INVENTION: Use of a class of enzymes and their encoding genes to increase oil content in transgenic organisms
   iii) Number of sequences: 2
2) INFORMATION FOR SEQ ID NO:1:
   i) SEQUENCE CHARACTERISTICS:
      A) LENGTH: 1833 bases
      B) TYPE: nucleic acid
      c) STRANDEDNESS: single
      D) TOPOLOGY: linear
   ii) MOLECULE TYPE: DNA
   iii) SEQUENCE DESCRIPTION:: SEQ ID NO: 1: 
ATGACGGAGA CTAAGGATTT GTTGCAAGAC GAAGAGTTTC TTAAGATCCG 50

CAGACTCAAT TCCGCAGAAG CCAACAAACG GCATTCGGTC ACGTACGATA 100

ACGTGATCCT GCCACAGGAG TCCATGGAGG TTTCGCCACG GTCGTCTACC 150

ACGTCGCTGG TGGAGCCAGT GGAGTCGACT GAAGGAGTGG AGTCGACTGA 200

GGCGGAACGT GTGGCAGGGA AGCAGGAGCA GGAGGAGGAG TACCCTGTGG 250
 
ACGCCCACAT GCAAAAGTAC CTTTCACACC TGAAGAGCAA GTCTCGGTCG 300

AGGTTCCACC GAAAGGATGC TAGCAAGTAT GTGTCGTTTT TTGGGGACGT 350

GAGTTTTGAT CCTCGCCCCA CGCTCCTGGA CAGCGCCATC AACGTGCCCT 400

TCCAGACGAC TTTCAAAGGT CCGGTGCTGG AGAAACAGCT CAAAAATTTA 450

CAGTTGACAA AGACCAAGAC CAAGGCCACG GTGAAGACTA CGGTGAAGAC 500

TACGGAGAAA ACGGACAAGG CAGATGCCCC CCCAGGAGAA AAACTGGAGT 550

CGAACTTTTC AGGGATCTAC GTGTTCGCAT GGATGTTCTT GGGCTGGATA 600

GCCATCAGGT GCTGCACAGA TTACTATGCG TCGTACGGCA GTGCATGGAA 650

TAAGCTGGAA ATCGTGCAGT ACATGACAAC GGACTTGTTC ACGATCGCAA 700

TGTTGGACTT GGCAATGTTC CTGTGCACTT TCTTCGTGGT TTTCGTGCAC 750

TGGCTGGTGA AAAAGCGGAT CATCAACTGG AAGTGGACTG GGTTCGTTGC 800

AGTGAGCATC TTCGAGTTGG CTTTCATCCC CGTGACGTTC CCCATTTACG 850

TCTACTACTT TGATTTCAAC TGGGTCACGA GAATCTTCCT GTTCCTGCAC 900

TCCGTGGTGT TTGTTATGAA GAGCCACTCG TTTGCCTTTT ACAACGGGTA 950

TCTTTGGGAC ATAAAGCAGG AACTCGAGTA CTCTTCCAAA CAGTTGCAAA 1000

AATACAAGGA ATCTTTGTCC CCAGAGACCC GCGAGATTCT GCAAAAAAGT 1050

TGCGACTTTT GCCTTTTCGA ATTGAACTAC CAGACCAAGG ATAACGACTT 1100
 
CCCCAACAAC ATCAGTTGCA GCAATTTCTT CATGTTCTGT TTGTTCCCCG 1150

TCCTCGTGTA CCAGATCAAC TACCCAAGAA CGTCGCGCAT CAGATGGAGG 1200

TATGTGTTGG AGAAGGTGTG CGCCATCATT GGCACCATCT TCCTCATGAT 1250

GGTCACGGCA CAGTTCTTCA TGCACCCGGT GGCCATGCGC TGTATCCAGT 1300

TCCACAACAC GCCCACCTTC GGCGGCTGGA TCCCCGCCAC GCAAGAGTGG 1350

TTCCACCTGC TCTTCGACAT GATTCCGGGC TTCACTGTTC TGTACATGCT 1400

CACGTTTTAC ATGATATGGG ACGCTTTATT GAATTGCGTG GCGGAGTTGA 1450

CCAGGTTTGC GGACAGATAT TTCTACGGCG ACTGGTGGAA TTGCGTTTCG 1500

TTTGAAGAGT TTAGCAGAAT CTGGAACGTC CCCGTTCACA AATTTTTACT 1550

AAGACACGTG TACCACAGCT CCATGGGCGC ATTGCATTTG AGCAAGAGCC 1600

AAGCTACATT ATTTACTTTT TTCTTGAGTG CCGTGTTCCA CGAAATGGCC 1650

ATGTTCGCCA TTTTCAGAAG GGTTAGAGGA TATCTGTTCA TGTTCCAACT 1700

GTCGCAGTTT GTGTGGACTG CTTTGAGCAA CACCAAGTTT CTACGGGCAA 1750

GACCGCAGTT GTCCAACGTT GTCTTTTCGT TTGGTGTCTG TTCAGGGCCC 1800

AGTATCATTA TGACGTTGTA CCTGACCTTA TGA 1833
2) INFORMATION FOR SEQ ID NO:2:
   i) SEQUENCE CHARACTERISTICS:
      A) LENGTH: 610 amino acids
      B) TYPE: amino acid
      D) TOPOLOGY: linear
   ii) MOLECULE TYPE: protein
   iii) SEQUENCE DESCRIPTION: SEQ ID NO: 2:

Met Thr Glu Thr Lys Asp Leu Leu Gln Asp Glu Glu Phe Leu Lys Ile
1               5                   10                  15

Arg Arg Leu Asn Ser Ala Glu Ala Asn Lys Arg His Ser Val Thr Tyr
            20                  25                  30

Asp Asn Val Ile Leu Pro Gln Glu Ser Met Glu Val Ser Pro Arg Ser
        35                  40                  45

Ser Thr Thr Ser Leu Val Glu Pro Val Glu Ser Thr Glu Gly Val Glu
    50                  55                  60

Ser Thr Glu Ala Glu Arg Val Ala Gly Lys Gln Glu Gln Glu Glu Glu
65                  70                  75                  80

Tyr Pro Val Asp Ala His Met Gln Lys Tyr Leu Ser His Leu Lys Ser
                85                  90                  95

Lys Ser Arg Ser Arg Phe His Arg Lys Asp Ala Ser Lys Tyr Val Ser
            100                 105                 110

Phe Phe Gly Asp Val Ser Phe Asp Pro Arg Pro Thr Leu Leu Asp Ser
        115                 120                 125

Ala Ile Asn Val Pro Phe Gln Thr Thr Phe Lys Gly Pro Val Leu Glu
    130                 135                 140

Lys Gln Leu Lys Asn Leu Gln Leu Thr Lys Thr Lys Thr Lys Ala Thr
145                 150                 155                 160

Val Lys Thr Thr Val Lys Thr Thr Glu Lys Thr Asp Lys Ala Asp Ala
                165                 170                 175

Pro Pro Gly Glu Lys Leu Glu Ser Asn Phe Ser Gly Ile Tyr Val Phe
            180                 185                 190

Ala Trp Met Phe Leu Gly Trp Ile Ala Ile Arg Cys Cys Thr Asp Tyr
        195                 200                 205

Tyr Ala Ser Tyr Gly Ser Ala Trp Asn Lys Leu Glu Ile Val Gln Tyr
    210                 215                 220

Met Thr Thr Asp Leu Phe Thr Ile Ala Met Leu Asp Leu Ala Met Phe
225                 230                 235                 240

Leu Cys Thr Phe Phe Val Val Phe Val His Trp Leu Val Lys Lys Arg
                245                 250                 255

Ile Ile Asn Trp Lys Trp Thr Gly Phe Val Ala Val Ser Ile Phe Glu
            260                 265                 270

Leu Ala Phe Ile Pro Val Thr Phe Pro Ile Tyr Val Tyr Tyr Phe Asp
        275                 280                 285

Phe Asn Trp Val Thr Arg Ile Phe Leu Phe Leu His Ser Val Val Phe
    290                 295                 300

Val Met Lys Ser His Ser Phe Ala Phe Tyr Asn Gly Tyr Leu Trp Asp
305                 310                 315                 320

Ile Lys Gln Glu Leu Glu Tyr Ser Ser Lys Gln Leu Gln Lys Tyr Lys
                325                 330                 335

Glu Ser Leu Ser Pro Glu Thr Arg Glu Ile Leu Gln Lys Ser Cys Asp
            340                 345                 350

Phe Cys Leu Phe Glu Leu Asn Tyr Gln Thr Lys Asp Asn Asp Phe Pro
        355                 360                 365

Asn Asn Ile Ser Cys Ser Asn Phe Phe Met Phe Cys Leu Phe Pro Val
    370                 375                 380

Leu Val Tyr Gln Ile Asn Tyr Pro Arg Thr Ser Arg Ile Arg Trp Arg
385                 390                 395                 400

Tyr Val Leu Glu Lys Val Cys Ala Ile Ile Gly Thr Ile Phe Leu Met
                405                 410                 415

Met Val Thr Ala Gln Phe Phe Met His Pro Val Ala Met Arg Cys Ile
            420                 425                 430

Gln Phe His Asn Thr Pro Thr Phe Gly Gly Trp Ile Pro Ala Thr Gln
        435                 440                 445

Glu Trp Phe His Leu Leu Phe Asp Met Ile Pro Gly Phe Thr Val Leu
    450                 455                 460

Tyr Met Leu Thr Phe Tyr Met Ile Trp Asp Ala Leu Leu Asn Cys Val
465                 470                 475                 480

Ala Glu Leu Thr Arg Phe Ala Asp Arg Tyr Phe Tyr Gly Asp Trp Trp
                485                 490                 495

Asn Cys Val Ser Phe Glu Glu Phe Ser Arg Ile Trp Asn Val Pro Val
            500                 505                 510

His Lys Phe Leu Leu Arg His Val Tyr His Ser Ser Met Gly Ala Leu
        515                 520                 525

His Leu Ser Lys Ser Gln Ala Thr Leu Phe Thr Phe Phe Leu Ser Ala
    530                 535                 540

Val Phe His Glu Met Ala Met Phe Ala Ile Phe Arg Arg Val Arg Gly
545                 550                 555                 560

Tyr Leu Phe Met Phe Gln Leu Ser Gln Phe Val Trp Thr Ala Leu Ser
                565                 570                 575

Asn Thr Lys Phe Leu Arg Ala Arg Pro Gln Leu Ser Asn Val Val Phe
            580                 585                 590

Ser Phe Gly Val Cys Ser Gly Pro Ser Ile Ile Met Thr Leu Tyr Leu
        595                 600                 605

Thr Leu
    610

### REFERENCES

Becker, D., Kemper, E., Schell, J., and Masterson, R. (1993) *Plant Mol Biol.* **20**, 1195-7
Bell, R.M., and Coleman, R.A. (1983) in *The Enzymes* (Boyer, P.D., ed.) Vol. 16, pp. 87-111, Academic Press, Orlando .
Bligh, E.G. and Dyer, W.J. (1959) *Can. J. Biochem. Physiol.* **37,** 911-917
Cao, Y.-Z., and Huang, A.H.C. (1986) *Plant. Physiol.* **82,** 813-820
Cases, S., Smith, S.J., Zheng, Y.-W., Myers, H.M., Lear, S.R., Sande, E., Novak, S., Collins, C., Welch, C.B., Lusis, A.J., Erickson, S.K., and Farese, R.V. (1998) *Proc. Natl. Acad. Sci.* **95**, 13018-13023
Dahlqvist, A., Ståhl, U., Lenman, M., Banas, A., Ronne, H., and Stymne, S. (1999) *Enzymes catalysing a transacylation reaction involved in triacylglycerol synthesis.* Presented at Biochem, Mol. Biol. Plant Fatty Acids Glycerolipids Symp., South Lake Tahoe, Calif., USA Dahlqvist, A., Ståhl, U., Lenman, M., Banas, A., Lee, M., Sandager, L., Ronne, H., and Stymne, S. (2000) *Proc. Natl. Acad. Sci. USA.* **97,** 6487-6492
Ekundayo, R.O. and Packter, N.M. (1994) *Microbiology* **140**, 931-943
Hobbs, D.H., Lu, C., and Hills, M.J. (1999) *FEBS Letters* **452**, 145-149
Lardizabal KD, Hawkins D, Thompson GA. (2000) International Patent No. WO 00/01713 Lardizabal, K., Hawkins, D., Mai, J., and Wagner, N. (1999) *Identification of a new diacylglycerol acyltransferase gene family.* Presented at Biochem, Mol. Biol. Plant Fatty Acids Glycerolipids Symp., South Lake Tahoe, Calif., USA
Lassner, M.W., Ruezinsky, D.M. (1999) International patent No. WO 99/63096
Martin, B.A. and Wilson, R.F. (1983) *Lipids* **18,** 1-6
Meesters, P.A.E.P., Huijberts, G.N.M., and Eggink, G. (1996) *Appl. Microbiol. Biotechnol.* **45,** 575-579
Oelkers, P., Behari, A., Cromley, D., Billheimer, J.T., and Sturley, S.L. (1998) *J. Biol. Chem.* **273,**26765-26771
Ratledge, C. (1989) in *Microbial Lipids* (Ratledge, C. and Wilkinson, S.G., eds.) 2, 567-668, Academic Press, London
Ronne, H., Carlberg, M., Hu, G.-Z., and Nehlin, J.O. (1991) *Mol. Cell. Biol.* **11,** 4876-4884 Sherman, F., Fink, G.R., and Hicks, J.B. (1986) Laboratory Course Manual for Methods in Yeast Genetics, Cold Spring Harbor Lab. Press, Plainview, NY
Stålberg, K., Ellerström, M., Josefsson, L.G., and Rask, L. (1993) *Plant Mol Biol.* **23,** 671-83 Ståhl, U. (1999) *Phospholipases and transacylases involved in triacylglycerol synthesis.* Presented at 23^{rd} World Congress and Exhibition of the International Society for Fat Research (ISF), Brighton, UK
Thomas, B.J. and Rothstein, R. (1989) *Cell* **56,** 619-630
Valvekens, D., van Lijsebettens, M., and van Montagu, M. (1992) in *Plant Tissue Culture.* (Lindsey K.), Kluwer Academic Publishers. NL
Yang, H., Bard, M., Bruner, D.A., Gleeson, A., Deckelbaum, R.J., Aljinovic, G., Pohl, T.M., Rothstein, R. and Sturley, S.L. (1996) *Science* **272**, 1353-1356
Yu, C., Kennedy, N.J., Chang, C.Y.G., and Rothblatt, J.A. (1996) *J. Biol. Chem.* **271,** 24157-24163

## Claims

1. A method for increasing the oil content of an oil-producing plant, comprising:
transforming said plant with a nucleotide sequence so that said organism expresses an enzyme that catalyzes the transfer of a fatty acid from acyl-CoA to diacylglycerol for the production of triacylglycerol (TAG), and wherein said enzyme comprises SEQ ID NO. 2.

2. The method according to claim 2, wherein said nucleotide sequence comprises SEQ ID NO. 1.

3. The method according to claim 2, wherein said nucleotide sequence is from a *saccharomyces cerevisiae* ARE1 gene.

4. The method according to any of claims 1-3, wherein said nucleotide sequence is transferred by recombinant DNA technology.

5. A transgenic plant, comprising a plasmid or genome containing a nucleotide sequence encoding an enzyme that catalyzes the transfer of a fatty acid from acyl-CoA to diacylglycerol for the production of triacylglycerol (TAG), wherein said nucleotide sequence is derived from SEQ. ID NO. 1 or *saccharomyces cerevisiae* ARE 1 gene, and said nucleotide sequence encodes an enzyme having an amino acid sequence comprising SEQ ID No. 2.

6. The transgenic plant according to claim 5, wherein said plant is an agricultural plant.

7. The transgenic plant according to claim 6, wherein said plant is an oil seed crop.

8. The transgenic plant according to any of claims 5-7, wherein said nucleotide sequence is expressed under the control of a storage organ specific promoter.

9. The transgenic plant according to claim 8, wherein said nucleotide sequence is expressed under control of a seed-specific promoter.

10. A method for increasing the oil content of an oil-producing organism, comprising:
transforming said organism selected from the group consisting of *Arabidopsis* and yeast with a nucleotide sequence comprising SEQ. ID NO. 1 or ARE 1 gene so that said organism expresses an enzyme and catalyzes the transfer of a fatty acid from acyl-CoA to diacylglycerol for the production of triacylglycerol (TAG), said enzyme comprising an amino acid sequence of SEQ ID No. 2.

11. A method for increasing the oil content of an oil-producing plant, comprising:
transforming said plant with a nucleotide sequence comprising SEQ. ID NO. 1 or ARE1 gene so that said organism expresses an enzyme and catalyzes the transfer of a fatty acid from acyl-CoA to diacylglycerol for the production of triacylglycerol (TAG), said enzyme comprising an amino acid sequence of SEQ ID No. 2.

## Patentansprüche

1. Verfahren zur Erhöhung des Ölgehalts einer ölproduzierenden Pflanze, umfassend:
Transformieren der Pflanze mit einer Nukleotidsequenz, so dass der Organismus ein Enzym exprimiert, das die Übertragung einer Fettsäure von Acyl-CoA auf Diacylglyerin katalysiert zur Herstellung von Triacylglyerin (TAG), wobei das Enzym SEQ ID NO. 2 umfasst.

2. Das Verfahren gemäß Anspruch 1, wobei die Nukleotidsequenz SEO ID NO. 2 umfasst.

3. Das Verfahren gemäß Anspruch 2, wobei die Nukleotidsequenz von einem *Saccharomyces cerevisiae* ARE1 Gen stammt.

4. Das Verfahren gemäß einem der Ansprüche 1 bis 3, wobei die Nukleotidsequenz mittels rekombinanter DNA-Technologie eingeführt wird.

5. Transgene Pflanze umfassend ein Plasmid oder Genom enthaltend eine Nukleotidsequenz, die für ein Enzym kodiert, das die Übertragung einer Fettsäure von Acyl-CoA auf Diacylglyerin katalysiert zur Herstellung von Triacylglyerin (TAG), wobei das Enzym von SEQ ID NO. 1 oder dem *Saccharomyces cerevisiae* ARE1 Gen abgeleitet ist, und die Nukleotidsequenz für ein Enzym mit einer Aminosäuresequenz umfassend SEQ ID NO. 2 kodiert.

6. Die transgene Pflanze gemäß Anspruch 5, wobei die Pflanze ein Agrikulturpflanze ist.

7. Die transgene Pflanze gemäß Anspruch 6, wobei die Pflanze eine Ölsaat ist.

8. Die transgene Pflanze gemäß einem der Ansprüche 5 bis 7, wobei die Nukleotidsequenz unter der Kontrolle eines speicherorganspezifischen Promoters exprimiert wird.

9. Die transgene Pflanze gemäß Anspruch 8, wobei die Nukleotidsequenz unter der Kontrolle eines samenspezifischen Promoters exprimiert wird.

10. Verfahren zur Erhöhung des Ölgehalts eines ölproduzierenden Organismus, umfassend: Transformieren des Organismus, der aus der Gruppe bestehend aus *Arabidopsis* und Hefe ausgewählt ist, mit einer Nukleotidsequenz umfassend SEQ ID NO. 1 oder das ARE1 Gen, so dass der Organismus ein Enzym produziert und die Übertragung einer Fettsäure von Acyl-CoA auf Diacylglyerin katalysiert zur Herstellung von Triacylglyerin (TAG), wobei das Enzym eine Aminosäuresequenz von SEQ ID NO. 2 umfasst.

11. Verfahren zur Erhöhung des Ölgehalts einer ölproduzierenden Pflanze, umfassend:
Transformieren der Pflanze mit einer Nukleotidsequenz umfassend SEQ ID NO. 1 oder das ARE1 Gen, so dass der Organismus ein Enzym produziert und die Übertragung einer Fettsäure von Acyl-CoA auf Diacylglyerin katalysiert zur Herstellung von Triacylglyerin (TAG), wobei das Enzym eine Aminosäuresequenz von SEQ ID NO. 2 umfasst.

## Revendications

1. Procédé permettant d'augmenter la teneur en huile d'une plante oléagineuse comprenant : la transformation de ladite plante avec une séquence de nucléotides de façon à ce que ledit organisme exprime une enzyme qui catalyse le transfert d'un acide gras de l'acyl-CoA sur le diacylglycérol pour la production de triacylglycérol (TAG) et dans lequel ladite enzyme comprend la séquence SEQ. ID N°2.

2. Procédé selon la revendication 1, dans lequel ladite séquence de nucléotides comprend la séquence SEQ. ID N°1.

3. Procédé selon la revendication 2, dans lequel ladite séquence de nucléotides provient d'un gène ARE1 de *saccharomyces cerevisiae.*

4. Procédé selon l'une des revendications 1 à 3, dans lequel ladite séquence de nucléotides est transférée à l'aide d'une technologie de recombinaison d'ADN.

5. Plante transgénique comprenant un plasmide ou un génome contenant une séquence de nucléotides codant une enzyme qui catalyse le transfert d'un acide gras de l'acyl-CoA vers le diacylglycérol pour la production de triacylglycérol (TAG), dans lequel ladite séquence de nucléotides est dérivée de la séquence SEQ. ID N°1 ou du gène ARE1 de *saccharomyces cerevisiae* et ladite séquence de nucléotides code une enzyme ayant une séquence d'acides aminés comprenant la séquence SEQ. ID N°2.

6. Plante transgénique selon la revendication 5, dans lequel ladite plante est une plante agricole.

7. Plante transgénique selon la revendication 6, dans lequel ladite plante est une culture à graine oléagineuse.

8. Procédé selon l'une des revendications 5 à 7, dans lequel ladite séquence de nucléotides est exprimée sous le contrôle d'un promoteur spécifique d'un organe de stockage.

9. Plante transgénique selon la revendication 6, dans lequel ladite séquence de nucléotides est exprimée sous le contrôle d'un promoteur spécifique à la graine.

10. Procédé permettant d'augmenter la teneur en huile d'un organisme oléagineux comprenant : la transformation dudit organisme, sélectionné dans le groupe constitué de *Arabidopsis* et d'une levure, avec une séquence de nucléotides comprenant la séquence SEQ. ID N°1 ou le gène ARE1 de façon à ce que ledit organisme exprime une enzyme et catalyse le transfert d'un acide gras de l'acyl-CoA vers le diacylglycérol pour la production de triacylglycérol (TAG), ladite enzyme comprenant une séquence d'acides aminés de SEQ. ID N°2.

11. Procédé permettant d'augmenter la teneur en huile d'une plante oléagineuse comprenant : la transformation de ladite plante avec une séquence de nucléotides comprenant la séquence SEQ. ID N°1 ou le gène ARE1 de façon à ce que ledit organisme exprime une enzyme et catalyse le transfert d'un acide gras de l'acyl-CoA vers le diacylglycérol pour la production de triacylglycérol (TAG), ladite enzyme comprenant une séquence d'acides aminés de SEQ. ID N°2.
